# EUROPEAN PATENT SPECIFICATION

(11) **EP 3 017 302 B1**
(45) Date of publication and mention of the grant of the patent: **24.04.2019**
(21) Application number: 14820113.0
(22) Date of filing: 03.07.2014
(51) Int. Cl.: G01N 33/50

(54) **METHODS FOR PREDICTING RESPONSES TO CHEMICAL OR BIOLOGIC SUBSTANCES**
VERFAHREN ZUR PROGNOSE VON REAKTIONEN AUF CHEMISCHE ODER BIOLOGISCHE STOFFE
PROCÉDÉ DE PRÉVISION DE RÉPONSES À DES SUBSTANCES CHIMIQUES OU BIOLOGIQUES

(30) Priority: 03.07.2013 US 201361842678 P
(43) Date of publication of application: 11.05.2016
(73) Proprietor: Coyne Scientific, LLC, Atlanta, GA 30339 (US)
(72) Inventor: COYNE, Kevin P., Atlanta, Georgia 30327 (US); COYNE, Shawn T., Smyrna, Georgia 30080 (US); LETCHWORTH,Sharon, Kernersville, North Carolina 27284 (US)
(74) Representative: Mewburn Ellis LLP
(86) International application number: PCT/US2014/045499
(87) International publication number: WO 2015/003178

(56) References cited:
- WO-A1-2009/035706
- WO-A2-00/58508
- GB-A- 2 471 389
- US-A- 5 175 004
- US-A1- 2009 220 996
- US-A1- 2009 306 680
- US-A1- 2012 196 285
- US-B1- 6 605 275
- US-B2- 8 017 397
- LAURA YLÄ-OUTINEN: "Human cell-based micro electrode array platform for studying neurotoxicity", FRONTIERS IN NEUROENGINEERING, vol. 3, no. 111, 30 September 2010 (2010-09-30), XP055320463, DOI: 10.3389/fneng.2010.00111
- YOAV MAYSHAR ET AL: "Teratogen screening using transcriptome profiling of differentiating human embryonic stem cells", JOURNAL OF CELLULAR AND MOLECULAR MEDICINE, vol. 15, no. 6, 17 June 2010 (2010-06-17), pages 1393-1401, XP055320568, RO ISSN: 1582-1838, DOI: 10.1111/j.1582-4934.2010.01105.x
- WEST P R ET AL: "Predicting human developmental toxicity of pharmaceuticals using human embryonic stem cells and metabolomics", TOXICOLOGY AND APPLIED PHARMACOLOGY, ACADEMIC PRESS, AMSTERDAM, NL, vol. 247, no. 1, 15 August 2010 (2010-08-15), pages 18-27, XP027142153, ISSN: 0041-008X [retrieved on 2010-05-21]
- ADLER ET AL: "First steps in establishing a developmental toxicity test method based on human embryonic stem cells", TOXICOLOGY IN VITRO, ELSEVIER SCIENCE, GB, vol. 22, no. 1, 19 December 2007 (2007-12-19), pages 200-211, XP022393683, ISSN: 0887-2333, DOI: 10.1016/J.TIV.2007.07.013
- ADLER SARAH ET AL: "Testing potential developmental toxicants with a cytotoxicity assay based on human embryonic stem cells", ATLA. ALTERNATIVES TO LABORATORY ANIMALS, LONDON, GB, vol. 36, no. 2, 1 May 2008 (2008-05-01), pages 129-140, XP009161023, ISSN: 0261-1929
- ANNE K KRUG ET AL: "Human embryonic stem cell-derived test systems for developmental neurotoxicity: a transcriptomics approach", ARCHIVES OF TOXICOLOGY, SPRINGER-VERLAG, BERLIN, DE, vol. 87, no. 1, 21 November 2012 (2012-11-21), pages 123-143, XP035157588, ISSN: 1432-0738, DOI: 10.1007/S00204-012-0967-3
- MEHTA A ET AL: "Assessment of drug induced developmental toxicity using human embryonic stem cells", CELL BIOLOGY INTERNATIONAL, ACADEMIC PRESS, GB, vol. 32, no. 11, 1 November 2008 (2008-11-01), pages 1412-1424, XP025574246, ISSN: 1065-6995, DOI: 10.1016/J.CELLBI.2008.08.012 [retrieved on 2008-08-20]

## Description

### FIELD OF THE INVENTION

The present application is directed to methods for predicting differential human responses to chemical and biologic substances using stem cells and more specifically relates to the use of extra-embryonic pluripotent or multipotent stem cells to predict and elucidate differential human responses to chemicals and biologic substances across a genetically diverse population.

### BACKGROUND OF THE INVENTION

For many years, scientists have known that genetic differences among humans play a major role in their individual reactions to various substances, including disease, allergens, biological agents, pharmaceuticals, and other chemicals. (Manry, et al., Cold Spring Harb. Perspect. Med. 2013; 3:a012450; Geeleher, et al., Genome Biology 15:R47 (2014)) However, scientists have been severely constrained in their ability to systematically analyze these differences.

For example, pharmaceutical drugs, industrial chemicals, biological agents and other compounds and substances with which human beings may come into contact, directly or indirectly, must be tested for safety and efficacy prior to public sale. Often, these drugs, chemicals, compounds and substances fail regulatory testing or clinical trials, or are recalled after introduction into the market, resulting in the loss of significant investments of time, effort and money by those who developed them.

Similar limitations have been observed when investigating the transmission of infectious diseases across humans of varied genetics. Epidemiology is limited to studying past occurrences, not potential ones. Case/control experiments are often inappropriate due to ethical issues of exposing healthy individuals to infectious diseases. Animal models do not reflect human genetic diversity. And, *in vitro* tests using classical cell based models are limited by either a restricted supply of cells from a single subject or a pool of tissue representing a small number of subjects (in the case of primary cell based assays), or by the potential for non-representative reactions (such as in tumor-based or engineered cell models).

In recent years, scientists have begun conducting *in vitro* experiments to test for the reaction to chemical or biological agents using pluripotent stem cells, especially human embryonic stem cells (hESCs) and human induced pluripotent stem cells (hiPSCs). These have proven to be valuable preliminary indicators of "human" responses to certain agents-be they chemical or biological. However, such stem cell-based tests have been used almost exclusively to provide an indication of a "typical" or "representative" human response - that is, tests have been run against hESCs or hiPSCs (or downstream derivatives or differentiations of such cells) from a single cell line, sourced from a single donor who, in effect, serves as a lone representative of the entire human race. These tests are not intended to detect any inter-donor variability in response.

Recently, scientists have created hiPSCs from patients, and relatives of those patients sharing a particular genetic feature, with a previously diagnosed condition or predisposition to react to a particular chemical agent, and used these hiPSCs to confirm that people who share a related genetic profile often share similar reactions (Manry, et al., (2013), *supra*).

However, none of the above approaches provide an investigation into variations in responses to a particular chemical or biological agent or substance across a genetically diverse population. Nor do these approaches enable the discovery of previously unrecognized variations in response or variations in the genetic causes of the response. As a consequence, these approaches fail to provide a way to discern previously unknown associations between genetic profiles and observed reactions.

Efficient *in vitro* tests are, therefore, needed to understand the distribution of human responses to chemical and biological substances.

### BRIEF SUMMARY OF THE INVENTION

Methods for predicting and elucidating differential human responses to chemical and biological substances, including but not limited to, pharmaceutical drugs, industrial chemicals, biologic agents (such as pathogenic bacteria or viruses, antibodies, proteins, DNA and its derivatives, RNA and its derivatives etc.), vaccines, or other compounds or agents, across a genetically and phenotypically diverse segment of the population, using extra-embryonic pluripotent or multipotent stem cells, are provided herein. The methods provided herein include both toxicity testing and (in the cases of pharmaceutical and vaccine testing) efficacy testing for all types of chemical and biological substances, as well as the study of diseases (including but not limited to, studies of susceptibility, transmission, virulence and gain of function), using all varieties of extra-embryonic pluripotent or multipotent stem cells.

In one embodiment the method is a test for differential responses among humans to toxicity responses to pharmaceutical compounds using extra-embryonic pluripotent stem cells obtained from neonate biological samples such as but not limited to, amniotic fluid-derived stem cells, which are obtainable using non-invasive or minimally invasive techniques from a variety of neonatal fluids including amniotic fluid, cord blood and neonatal urine. In summary, pluripotent or broadly multipotent extra-embryonic stem cells are isolated from a readily available biological fluid source, such as amniotic fluid, from large numbers (N > 20) of newborns who have been carefully selected and screened to match exacting criteria, and to represent a particular genetically diversified population. The cells are expanded, and if necessary, differentiated into functional cell types, under tightly defined procedures that greatly minimize known sources of process variation between copies of cells from the same donor and/or between donors. Validated pharmaceutical compounds from active, inactive, moderate and high toxicity classes are applied to these stem cells (or their derivatives) from the donors; cells that are sufficiently responsive to the validated chemicals are judged to be good indicators of toxic response; and there are sufficiently different responses among the cells from the different donors tested to indicate that the cells are useful as satisfactory indicators with respect to the differential human toxicity responses across a genetically diverse population.

In accordance with the method of predicting toxic reactions among genetically diverse populations, extra-embryonic pluripotent or broadly multipotent stem cells of large numbers (minimally twenty, preferably three hundred, or more preferably thousands) of genetically diverse donors, are isolated. Exemplary genetically diverse donors include diverse populations, such as donors of both genders and donors of multiple races. The stem cells can be isolated from a variety of sources, such as, but not limited to, amniotic fluid. The cells of a given donor are isolated and expanded in a laboratory setting using methods known by those skilled in the art for the particular cell chosen such as the process described in US Patent 7,569,385 B2. At the time of collection, phenotypic and other identifiers of the donor are recorded and each cell line may be assigned an identifying number for comparison tests in the future. The cell lines are subsequently expanded via population doublings/passaging using standard industry protocols, but with uniquely tight controls over the processes, equipment, and number of passages in order to minimize any sources of variation other than the genetic profiles of the cells themselves. The cell lines are then preserved, such as by cryopreservation.

The cell lines are subsequently thawed and passaged again using standard industry protocol, and the cell lines from large numbers of different donors are used to gauge toxicity responses, for example by exposing cells from a given cell line to a given drug at a given concentration for a given length of time, then adding biomarkers that indicate end points of interest (such as, but not limited to, cell viability), and plotting data regarding what portion, if any, of the donor population experienced reactions of different magnitudes than others. Differential reactions may be measured along any of several dimensions using any of several techniques. For example, cytotoxicity may be assessed via one or more staining tests.

The various toxicity responses of cell lines from large numbers of donors are analyzed, and the differential responses among the tested cell lines are analyzed, to provide a variety of useful data, including, but not limited to, the total percentage of the representative human population likely to experience an adverse reaction, or the key phenotypic characteristics, for example, gender or race, of those individuals within the represented human population that are most likely to experience an adverse reaction.

Finally, individuals are optionally grouped into sub-cohorts based on quantitative ranges of reaction, and gene association analyses are conducted to determine whether specific gene alleles, or combinations of gene alleles, are statistically associated with such differences in reaction.

### DETAILED DESCRIPTION OF THE INVENTION

Described herein are methods to predict human responses to chemical and biological substances, including, but not limited to pharmaceutical drugs, industrial chemicals, or other compounds, molecules, biologic agents (such as pathogenic bacteria or viruses, antibodies, proteins, DNA and its derivatives, RNA and its derivatives, etc.), or vaccines, across a genetically and phenotypically diverse segment of the population using all varieties of extra-embryonic pluripotent or multipotent stem cells.

### Definitions

The terms "extra-embryonic stem cell" or "extra-embryonic pluripotent or multipotent stems cells" are defined herein as any pluripotent or multipotent stem cell that is not an embryonic stem cell, but that is a cell of the fetus/newborn (as defined by having the same DNA as the fetus/newborn) and that can be found in any one or more organs of a fetus/newborn while in utero or in the fetus/newborn-centric tissues and fluids (including, but not limited to, amniotic fluid, the placenta, chorionic villus of the placenta, the umbilical cord, cord blood, and the amniotic sac) regardless of whether that stem cell is collected during gestation or after birth. The term, as defined herein, also specifically includes any stem cell found in the urine of a baby that is excreted within three days of birth.

The term "compare-and-contrast statistical test" is defined herein as any mathematical or statistical test that draws inferences about the relative behavior or cause of relative behavior of any cohort or sub-cohort as compared to the behavior or cause of behavior of any other cohort or sub-cohort in a stimulus-response experiment. This definition specifically includes, but is not limited to: comparison of arithmetical means, medians, and ranges; Genome Wide Association Studies; exome or other partial-genomic association studies; and comparisons of the gene expression or proteomic expression of cohorts and sub-cohorts.

The term "inter-donor" is defined herein as the distinction between one donor and another donor. The phrase may refer to the donors themselves, or to cells taken from or derived from cells taken from such donors.

The term "intra-donor" is defined herein as the distinction between the group of cells from a single donor that participate in a single copy of an experimental procedure, and other groups of cells from that same donor that participate in either other copies of the same experiment, or other experiments.

The term "cross-replicate" is defined herein as a comparison of results across intra-donor groups of cells within a single experiment.

The term "cross-donor" is defined herein as a comparison of results between the cells of one donor and those of another donor.

The term "pluripotent" as used herein is defined to mean a stem cell that can differentiate into any of the three germ layers (endoderm, ectoderm and mesoderm).

The term "multipotent" as used herein is defined to mean a stem cell that can differentiate into multiple, but limited, cell types.

### Method overview

A robust *in vitro* platform combining an extensive supply of cells from a large number of donors who collectively represent the genetic diversity of a population of interest (e.g. the U.S. population), with extremely consistent processes for cell production, test administration, and post-test analysis, is provided herein to open important new vistas of scientific understanding.

For example, in the case of toxicity testing of chemicals such as pharmaceuticals, certain potentially negative but relatively infrequently-occurring outcomes associated with pharmaceutical drugs, industrial chemicals, and other compounds and substances can be identified via *in vitro* testing. This will enable persons or institutions attempting to create, manufacture or distribute pharmaceutical drugs, industrial chemicals, and other compounds and substances with which human may come into contact, to save time, effort and money. In addition, this will significantly benefit society at large to be better protected from harm, and to enable a larger number of new and valuable substances to come to market more quickly at lower prices.

As a second example, in the case of disease investigation, the present methods provide scientists with a repeatable tool that can be used to directly examine the differences in transmission rates of mutations of an infectious disease. The tools are useful for detecting whether some populations are more vulnerable to a new disease than others. They can be used to directly analyze differences in gain-of-function as mutations of a disease pass through multiple generations of victims. And, they can be utilized to learn about the relative effectiveness of vaccines and/or treatments in various sub-populations more efficiently than is possible with conventional *in vitro* platforms.

The development of a robust *in vitro* platform involves far more than simply assembling samples of cells from a large number of donors and repeating an experiment on each of them, because the variation in response to a particular chemical or biological substance due to genetic differences is often relatively small for most participants in a study (See Harrill, et al., Genome Research 2009:1507-1515 (2009)), and the causes of extraneous variation are numerous. Thus, many extra steps and protocols are required to ensure that the "noise" of such extraneous sources does not hide the "signal" from the genetic differences.

Thus, a robust platform to carry out such *in vitro* investigations must meet two criteria that are simple in concept, but extremely difficult to put into practice: (1) that the only source of significant variation in reactions between donors in the same experiment must be the differences in the genetic profiles of the donors, and (2) that the only source of significant variation in reactions between two identical experiments involving the same donor but different substances must be the nature of the substances.

Thus, the ability to discern the role of genetics in determining the variation of responses to a chemical or biological substance for a diversified population requires combining a number of novel approaches to various elements of the platform, including novel selection of a suitable cell type, stringent selection rules for donors in a cohort, consistency of production processes and statistical clustering techniques as explained in more detail below.

### Novel selection of a suitable cell type

hESCs and hiPSCs have been the best known and most widely used stem cells to date. However, neither is suitable for method described herein.

Ethical issues alone prevent the development of a sufficient number of hESC cell lines to support a large sample of a broadly genetic diverse population. In addition, hESC cell lines are expensive to produce. Further, given the source of the embryos (i.e. discarded embryos from *in vitro* fertilization procedures), it is not always possible to obtain phenotypic information on the donors, such as family history.

hiPSCs present a number of issues. First, while most scientists accept that the single largest driver of differences among subjects in response to an agent is their differences in their genetic profiles, two other factors the age of the subject, and the environments to which the subjects have been exposed throughout their lifetimes also play a role, thus potentially disguising the role of genetics. hiPSCs induced from donors of different ages embed their unique age-specific effects (such as shortened telomeres), as well as each donor's individual history of environmental exposure (including, but not limited to: pollution, direct chemical exposures, history of diseases and illnesses, and dietary habits), thus clouding the ability to discern genetics-only effects. In addition, the process of inducing the donors' cells to pluripotency involves inserting genetic material into the cell thus potentially contaminating the very genome that is to be studied. In addition, the inducement process is lengthy and complex, presenting many "extra" opportunities for introducing extraneous sources of variation among the cells from separate donors due to unnoticed variations in process from one donor to another. These limitations are in addition to the low yield rates, high failure rates, and consequently high expense currently associated with developing a new hiPSC line.

Extra-embryonic pluripotent or multipotent stem cells are derived from tissues or fluids other than the embryo that are associated with the gestation and birth of a baby, are already pluripotent or broadly multipotent at the time of collection and isolation, and avoid the above issues. They are ethically non-controversial in that an embryo does not need to be destroyed to collect the cell, are of identical age for all donors (if selected from tissues taken immediately post-birth), and all donors have been exposed to similar environments (i.e. the pre-natal environment only). They do not require genetic manipulations to achieve pluripotency or multipotency, thus avoiding the potential for DNA contamination or process-based variation.

Although numerous publications have listed "drug screening" as a potential use of extra-embryonic stem cells, none have described the actual methods necessary. Therefore, the present methods represents the first use of extra-embryonic cells in experiments to test the impact of chemical or biological substances, and by definition, their use for testing such impacts across the broad range of human genetic diversity.

### Stringent selection rules for donors in a cohort

The design of the cohort of stem cell donors is central to any conclusions about the role of genetic diversity. For example, the minimum size of a randomly selected cohort must correspond to the level of precision being sought. For example, a cohort size of at least approximately 300 is minimally necessary to achieve a 95 percent probability of observing at least one instance of an effect (or underlying genetic pattern) that has a prevalence of 1 percent in the population of interest. As another example, a cohort size of at least approximately 20 is minimally necessary to achieve a 95 percent probability of observing at least one instance of an effect (or underlying genetic pattern) that has a prevalence of 14 percent in the population of interest.

Further, each member of the cohort must be selected in a way that maximizes the standardization among donors of all factors other than genetics that are known to have an impact, or even suspected of potentially having an impact on a donor's reaction to the chemical or biological substance under investigation. For example, differences in the pre-natal environment from a potential donor's mother's use of alcohol, drugs or tobacco would be reasons for exclusion from the cohort. In addition, while complete control of the mothers' external environment (such as differential exposure to pollution) is not possible, narrowing the sourcing to a single city or region that is known to have not experienced severe or unusual pollution issues (such as nuclear waste contamination, major chemical spills, water supply contamination, etc.) during a deliberately chosen narrow window of time for the gestation and birth of all donors improves the chances that interference from external environmental exposure is minimal.

### Consistency in production process

Extremely high consistency in the production processes is needed (1) across replicates of a single donor within an experiment, (2) across donors within a single experiment, and (3) between identical donors across multiple experiments. Under normal circumstances in biological research, protocols are designed separately for each experiment. To achieve the level of consistency required in the present methods, many of the protocols must be established and standardized for all experiments in advance. It is well known to those skilled in the art that standard operating procedures are routinely used in biological research.

For example, to achieve high consistency in production across replicates of a single donor within an experiment ((1) above), all cells from a single donor used in all experiments within the set should have been created at approximately the same time-without separation into sub-populations during the expansion process, and all experiments within a set should be carried out using substantially identical protocols on the same or equivalent robotic equipment, preferably using substantially identical reagents.

To achieve high consistency in production across donors within a single experiment ((2) above), the number of population doublings through which every donor has been passaged during the cell expansion process must be held within a narrow range across every donor, as scientists have found that the number of population doublings/passages can affect both the genome and the responsiveness of extra-embryonic stem cells. (Chen, WQ et al., J Proteome Res.8: 5285-95 (2009)).

To achieve high consistency in production between identical donors across multiple experiments ((3) above), all experiments within a set must be carried out using substantially identical protocols on the same or equivalent robotic equipment, preferably using substantially identical reagents and all cells from a single donor used in all experiments within the set must have been created at approximately the same time-without separation into sub-populations during the expansion process.

### Statistical Clustering Techniques

The application of statistical clustering techniques including those new to the field of Genome Wide Association Studies (GWAS) and other genomic analysis methods are useful techniques to utilize in the methods provided herein. It is well documented that the linkage between differential reactions to chemical or biological agents and differences in the genomic profile is often the result of multiple genes acting in various combinations. However, to date, because the only source of data to support the investigation of the relationships between reactions and broad genetic diversity has been clinical data (which is subject to many sources of "noise", and can therefore only be relied upon for the most basic distinctions) genomic studies (such as Genome Wide Association Studies) searching for the alleles that underlie differential reactions have been limited to a scheme that bifurcates subjects (analogous to donors) into Cases versus Controls. Given the potential for much more precise measurements of the degree of reaction in the present invention, it enables the reliable division of donors into any number of sub-cohorts based on their degree of reaction along a single measure, or even based on their degrees of reaction to multiple measures simultaneously. This opens the door to the application of many statistical techniques that have not been used in this field before.

### Methods

Embodiments of the methods provided herein preferably contain the following steps, namely: collecting, isolating, and expanding collecting a sample of extra-embryonic pluripotent or multipotent stem cells from a large number of specified donors; defining a set of end point parameters and end point indicators for measuring the various levels of chemical or biological substance response; subjecting the cells to the chemical or biological substance under investigation; adding reagents or biomarkers (or using other testing mechanisms) to test the cells; and comparing results on a donor-to-donor basis to determine what percentage of donor samples reach the threshold of interest. Each step of an embodiment of the methods provided herein is described in more detail as follows.

### Sample collection, isolation and expansion

The necessary volumes of extra-embryonic pluripotent or multipotent stem cells from a large number of donors of interest are obtained. While the methods provided herein include use of all varieties of extra-embryonic pluripotent or multipotent stem cells, for illustration purposes the methods are described in this embodiment using amniotic fluid-derived pluripotent stem cells.

In a preferred embodiment, the minimal number of donors is approximately 20; more preferably approximately 300; and most preferably approximately 1,000 or more. As the number of donors in the sample bank increases, the testing is better able to predict the prevalence of adverse reactions, even relatively rare reactions, in the broader population. For example, as previously noted, a random sample of 300 donors will have a 95 percent probability of containing at least one instance of any phenomenon (including, in this case, the tendency to an adverse reaction to the substance being tested) that has a prevalence of one percent or more in the population being sampled.

Donors are selected to build a stratified sample of the human population, by (1) defining the intersections of all externally discernible phenotypes that have been associated with differences in toxicity reactions to pharmaceutical compounds. In this embodiment, the phenotypes are limited to gender and race. (See Wenwi, et al., J Biochem Molecular Toxicology, 27:17-25 (2013).); (2) subdividing the total sample size, in a scheme that enables sufficient statistical representation within each sub-cohort; and (3) developing a stringent set of selection/exclusion criteria to reduce and remove known and suspected sources of variation other than genetic differences. Specifically, donors are excluded by eliminating those donors having the following:
1. Any donor with one or more known genetic abnormalities.
2. Evidence of newborn distress requiring intervention prior to or during fluid collection (respiratory interventions such as suction and oxygen delivery are acceptable).
3. Mother, fetus or newborn that undergoes an experimental procedure or exposure to experimental product or ionizing radiation prior to delivery.
4. Fetal demise or major fetal anomalies.
5. Infants born with birth defects that in the opinion of the investigator, may complicate the suitability of the amniotic fluid to contain stem cells (such as renal impairment).
6. Infants born with birth defects that may impede the flow of urine through the urethral meatus (such as a bladder outlet obstruction).
7. Gestation not between 35-39 weeks of pregnancy.
8. Mother who is unwilling to disclose any and all use of medication/drugs/alcohol/tobacco or nicotine products during pregnancy.
9. Complicated pregnancy (evidence of birth defects or chromosomal anomalies, pre-term labor, etc.).
10. Major maternal medical illness associated with increased risk for adverse pregnancy outcome (for example, any diabetes mellitus including gestational diabetes, lupus, any hypertensive disorder including gestational hypertension or preeclampsia, cardiac disease, renal insufficiency).
11. Evidence of maternal infection with communicable disease such as HIV, hepatitis, meningitis, gonorrhea, syphilis or any disease that the investigator believes may transfer to the infant.
12. Presence of Category A infectious diseases, and/or the presence of, but not limited to, HIV, hepatitis, adenovirus, herpes simplex virus, Epstein Barr virus, streptococcal bacteria.
13. Maternal history of blood transfusions or receipt of blood products, including rhogam.
14. Evidence of low amniotic fluid (oligohydramnios) in this pregnancy (the pregnancy used to collect the fluid sample), defined as less than 5 cm on the amniotic fluid index requiring intervention.
15. Use of prescription drugs during pregnancy (exception for antiemetics for nausea and vitamin supplements, and for the delivery of the infant (pain medication, antibiotics, etc.).
16. Use of investigational drugs not yet approved by the FDA during pregnancy.
17. Consumption of drugs of abuse during pregnancy, including psychomotor stimulants, hallucinogens, opiates, marijuana, designer drugs, alcohol, tobacco or nicotine products.
18. Birth mother is known to have been exposed to any biological or chemical hazard beyond the threshold known to cause chromosomal damage, birth defects, or chronic health impairment.

Further, subject to the requirement to reach the numbers required of each sub-sample, the geographic location and the timing of the births are compressed to the maximum degree practical in order to minimize any differences in pre-natal environment due to external pollution, etc. Further, the collection methods, tissue preservation methods (if necessary), and transportation methods are rigidly standardized across all donors.

The amniotic fluid-derived stem cells of a given donor are isolated and expanded in accordance with methods known to those skilled in the art, such as the methods described in US Patent 7,569,385 B2. However, additional requirements are imposed to prevent even typical levels of variation in processing from donor to donor. For example, all procedures for all donors are preferably carried out using substantially identical robotic equipment, and substantially identical reagents. In addition, rather than adjust protocols as necessary to accommodate individual variation on cell behavior across donors, donors are eliminated from the cohort and replaced if the protocol fails to produce successful results for that particular donor. Cell viability is then measured, for example, by trypan blue exclusion staining or other viability methods, once the cells are initially passaged.

Optimal cell expansion is preferably achieved as follows:
1. Conducting each expansion operation on the same piece of automated equipment using the same protocol for each donor as for every other donor.
2. Controlling the environment in which that robotic operation is conducted to be similar for every donor through one or more of the following:
   a. Enclosing the equipment in biological cabinets that include HEPA air filtration;
   b. Controlling the temperature within a specified range;
   c. Controlling the humidity within a specified range;
   d. Controlling the atmospheric composition within a specified range;
   e. Controlling the sources of, wavelength of, and intensity of, light exposure within a specified range;
   f. Controlling the noise level to which the cells are exposed within a specified range;
   g. Controlling the presence of any electrical or magnetic field to which the cells are exposed within a specified range.

It is known by those skilled in the art that extra-embryonic stem cells have been known to "drift" in both their genetic composition and in their reactivity levels as the number of generations through which the cells have been expanded increases. Therefore, in addition to the standard protocol provided herein for cell expansion (including the same level of extra process controls required to ensure cross-donor consistency described for the isolation and initial passaging stages described above) the process preferably includes ensuring that the population-doubling generation number is highly consistent across all donors by:
1. Calculating the minimum number of cells from each member of the cohort necessary to conduct all planned experiments,
2. Calculating the target number of population doublings required to reach the minimum number of required cells, assuming that the expansion process for each donor begins with a single cell,
3. Originating the expansion process for each donor from a single cell,
4. Expanding the population of each cell line until either of the following two conditions has been met: the necessary number of cell has been reached, or the "target" number of doublings has been exceeded by two. The actual number of doublings required to meet the two conditions must be tracked, and
5. Eliminating from the cohort any donor that fails to reach the necessary number of cells within the target number of population doublings plus two.

In addition, to ensure that the population doubling has not resulted in genetic drift that would provide misleading results, the entire genome of each donor is fully sequenced (preferably utilizing an automated sequencer such as, for example, the Ion Proton Sequencer from Life Technologies, Carlsbad, CA), and the resulting genome is compared to the Human Reference Genome (See "E. pluribus unum", Nature Methods 331 (October 2010).) Any donor whose genome fails to align is removed from the cohort and replaced.

Each donor is optionally assigned a number for identification purposes. Identification allows researchers to discern race, gender and family medical history of donors for use in comparison studies later. Parents of donors may also grant researchers permission to contact them in the future to check up on personal medical history of the donor, etc.

The extra-embryonic stem cells may be differentiated into any of several "downline" or "downstream" cell types (such as, but not limited to, embryoid bodies and other three-dimensional structures, cardiomyocytes, hepatocytes, and/or neurons) using one or more validated differentiation techniques well known to those skilled in the art.

Optimal differentiation is preferably achieved as follows:
1. Conducting each differentiation operation on the same piece of automated equipment using the same protocol for each donor as for every other donor.
2. Controlling the environment in which that robotic operation is conducted to be similar for every donor through one or more of the following:
   a. Enclosing the equipment in biological cabinets that include HEPA air filtration
   b. Controlling the temperature within a specified range
   c. Controlling the humidity within a specified range
   d. Controlling the atmospheric composition within a specified range
   e. Controlling the sources of, wavelength of, and intensity of, light exposure within a specified range
   f. Controlling the noise level to which the cells are exposed within a specified range
   g. Controlling the presence of any electrical or magnetic field to which the cells are exposed within a specified range.
   h. Aliquoting the cells of each donor as appropriate for the experiments to follow, and consistently cryo-preserving all aliquots from all donors, thereby subjecting all experimental units to the identical number of freezings and thawings.

### Defining end point parameters and end point indicators for measuring various levels of response

Threshold points are defined in order to determine which donor samples have significant reactions to the compound being tested. Knowing where a particular donor falls in the given spectrum allows for later examination of inter-donor variability. For example, based on results, comparison studies are optionally performed to examine the impact of race, gender, or genetic and/or epigenetic traits on the likelihood of a donor experiencing a toxic reaction of a particular degree. Further, a comparison study is optionally used by taking two populations of outliers - for example, one that exhibits the lowest 10% reaction to a given drug and another that exhibits the highest 10% reaction to a given drug - and examining differences between them. Such comparisons are useful to help researchers understand what factors put different segments of the population at risk for certain adverse reactions.

### Exposure to chemical or biological substance under investigation

The cells, whether basic stem cells or any of "downline" derivatives thereof, are cultured, such as by plating onto microtiter assay plates, then exposed to the chemical or biological substance of interest for a predetermined period, such as, but not limited to, three days. Of note, the results for the compounds of interest are calibrated by comparing against the results of internal standards with known toxicities, such as, but not limited to, 5-FU, saccharin, ATRA, hydroxyurea, 13-cis-RA, or other standard substances. The same vehicle (for example, DMSO) is preferably used to dissolve all chemical or biological substances to keep variables to a minimum.

Cultured cells are tested at multiple concentrations (preferably seven), and replicates are performed. During the duration of the study, cells are maintained in culture using standard cell culture procedures such as replacing growth media periodically after several days with fresh media containing corresponding growth factors and the requisite dosage of the compound being tested to maintain constant exposure levels.

All of the above steps are preferably performed using robotic fluid handling machines, incubators, etc., such that every process step is conducted substantially identically for each replicate of each donor, and for every donor as for every other donor. These include, but are not limited to: the sequence of each process step; the specific actions to be taken; the timing of, and timing between each step; and the specific reagents to be employed.

### Addition of testing mechanisms

Testing mechanism, such as reagents or biomarkers, are added to the cells to test for various adverse effects (such as, but not limited to, mitochondrial activity, or damage to the cell wall). Alternatively, other established tests are conducted such as, but not limited to, Multi Electrode Array analysis of cardiomyocytes.

Results are then read by the appropriate tools such as, but not limited to, a quantitative plate reader such as the GE Incell 2200 (GE Healthcare, Pittsburgh, PA).

All steps are most preferably conducted using automated means such as robotic fluid handlers and other robotic equipment such as, for example, the Tecan Freedom Evo 150 (Tecan US, Morrisville, NC).

### Comparing results on a donor-to-donor basis

Knowledge of how certain substances test in relation to substances of a similar class allows useful comparisons (for example, one statin can be compared to a variety of other statins to see if it is more or less harmful than comparable drugs to certain donor samples, while being less harmful to others).

Results are compared on a donor-to-donor basis using multiple statistical techniques.

### Preferred embodiments of the present methods

In one preferred embodiment of the methods provided herein, pharmaceutical drugs, industrial chemicals, or other compounds or substances are screened for various measures of human embryo toxicity or developmental toxicity, and to predict and elucidate differential toxicity responses among different donors based on their genetic diversity.

One fundamental test of human toxicity in pharmaceutical drugs is to determine whether an embryo is viable and will still differentiate, as it normally does, into three germ layers, despite being in the presence of a pharmaceutical compound being tested.

Testing is performed by differentiating one or more varieties of extra-embryonic pluripotent or multipotent stem cells into embryoid bodies using any of several industry standard protocols, then administering the candidate compound to the resulting embryoid bodies for a prescribed incubation period. Biomarkers are then used to determine whether all three germ layers are present. Presence of all three germ layers indicates that the tested compound is not harmful; whereas absence of one, two, or all three germ layers indicates a toxic effect. Cell survival and/or cell death is optionally assessed to determine viability of the embryoid body.

The screening method enables analysis of toxicity results within embryoid bodies across genetically diverse donors, allowing the drug manufacturer to: (1) identify adverse toxicity reactions within embryoid bodies even if they occur with very low incidence (for example, in only 1% or 2% of the population); (2) determine whether to continue or stop development of the candidate drug; and (3) understand the differences between those donors who experienced an adverse reaction and those who did not, which is useful for assisting the drug manufacturer in the modification of the compound to eliminate the adverse reaction, and/or to appropriately select or monitor specific patients for clinical trials, and/or to develop an appropriate warning that the candidate drug should not be administered to patients with a certain genetic profile.

In another embodiment, tissue-specific toxicity testing of pharmaceutical drugs, industrial chemicals, or other compounds or substances, using the methods provided herein, are used to predict and elucidate differential toxicity responses among different donors based on their genetic diversity.

As mentioned above, the methods described herein utilize either basic, undifferentiated or "upline" extra-embryonic pluripotent or multipotent stem cells or utilize any of several "downline" cell types into which the extra-embryonic pluripotent or multipotent stem cells may be differentiated using one or more well-validated differentiation techniques. Exemplary differentiated cells include, but are not limited to, cardiomyocytes, hepatocytes, osteoblasts, chrondroblasts, myocytes, epithelial cells, liver cells, pancreatic cells, neurons, embryoid bodies or other three-dimensional structures.

This is beneficial because differentiation to specific cell types enables researchers to know whether or not the compound being tested exhibits toxic effects to specific tissues or organs of the body. In this way, pharmaceutical drugs, industrial chemicals, and other compounds and substances with which human may encounter directly or indirectly can be tested for toxicity by various cell types across genetically diverse donors.

For example, as alluded to above, extra-embryonic pluripotent or multipotent stem cells are differentiated into cardiomyocytes to test for cardiotoxicity of pharmaceutical agents. Toxicity is assessed using any of several staining tests, and/or via testing using micro-electrode arrays or other cardiac ion channels to study differences in electrical activity or beat rate. Given that there are known genetic variants in cardiac ion channels, and many yet to be identified, pharmaceutical agents (or other compounds or substances) are tested for adverse toxicity reactions over a genetically diverse population sample to determine if they occur even with very low incidence.

As another example, Parkinson's disease is characterized by loss of dopaminergic neurons in the brain and has known genetic and environmental risk factors. Several genes have been associated with increased risk of Parkinson's disease (alpha-synuclein, Parkin, DJ1, Pink1, LRRK2, etc.) and familial cases for which a genetic basis has not yet been determined. In addition, agricultural chemicals such as rotenone, paraquat and maneb are toxic to dopaminergic neurons and are risk factors for developing Parkinson's disease. Thus, extra-embryonic pluripotent or multipotent stem cells differentiated into dopaminergic neurons from a panel of donors with genetic risk factors or familial history of Parkinson's disease, with or without donors with no familial link to Parkinson's disease for comparison, are tested for increased toxic reaction to industrial chemicals currently on the market or in development. This enables the manufacturer to better understand the health hazards of their products and include the appropriate warnings in the product insert or on the product label.

The methods provided herein enable analysis of pharmaceutical drugs, industrial chemicals, and other compounds and substances for toxicity results within specific cell types across genetically diverse donors, allowing the manufacturer to: (1) identify adverse toxicity reactions in specific cell types even if they occur with very low incidence (for example, in only 1% or 2% of the population); (2) determine whether to continue or stop development of the candidate drug; and (3) understand the differences between those donors who experienced an adverse reaction and those who did not, which is useful for helping the drug manufacturer to modify the compound and eliminate the adverse reaction, and/or to appropriately select or monitor specific patients for clinical trials, and/or to develop an appropriate warning that the candidate drug should not be administered to patients with a certain genetic profile.

In yet another embodiment, the present methods are used for tissue-specific efficacy testing of pharmaceutical drugs or other compounds or substances to predict and elucidate differential efficacy responses among different donors based on their genetic diversity.

Again, the methods described herein utilize either basic/upline extra-embryonic pluripotent or multipotent stem cells or any of several downline cell types into which the extra-embryonic pluripotent or multipotent stem cells may be differentiated (including, but not limited to, cardiomyocytes, hepatocytes, osteoblasts, myocytes, chondrocytes, epithelial cells, liver cells, pancreatic cells, neurons embryoid bodies or three-dimensional structures) using one or more well-validated differentiation techniques.

This is beneficial because differentiation to specific cell types enables researchers to know whether or not the compound being tested exhibits efficacious effects within specific tissues or organs of the body. In this way, pharmaceutical drugs, and other compounds and substances can be tested for efficacy of various cell types across genetically diverse donors.

For example, the ultimate goal of developing treatments for neurodegenerative diseases, such as Alzheimer's disease and Parkinson's disease, is to slow, halt or reverse the progression of the disease. Thus far, most clinical trials for neurodegenerative diseases have yielded disappointing results, mostly due to inconsistent efficacy across the clinical trial population. Since neurodegenerative diseases result from either single genetic risk factors, interactions between multiple genetic risk factors, or interactions between genetic and environmental factors, then variable patient response is to be expected. While some of these risk factors have been identified, many of them are yet to be identified.

As described above, Parkinson's disease is characterized by loss of dopaminergic neurons in the brain and has known and unknown genetic and environmental risk factors. Several genes have been associated with increased risk of Parkinson's disease (alpha-synuclein, Parkin, DJ1, Pink1, LRRK2, etc.), and with familial cases for which a genetic basis has not yet been determined. Thus, extra-embryonic pluripotent or multipotent stem cells differentiated into dopaminergic neurons from a sample of donors with genetic risk factors or familial history of Parkinson's disease as well as donors with no familial link to Parkinson's disease, are tested (with or without the presence of known dopaminergic toxins) for increased efficacy response, such as dopaminergic phenotypic expression (for example, tyrosine hydroxylase expression) or increased cell survival. This enables the manufacturer to better understand the patient population that is likely to be responsive to the substance being tested.

The methods described herein enable analysis of pharmaceutical drugs and other compounds and substances for an efficacious response within specific cell types across genetically diverse donors, allowing the manufacturer to: (1) identify efficacy in specific cell types and determine if a portion of the donor sample is less-responsive or non-responsive, even with very low incidence (for example, in only 1% or 2% of the population); (2) determine whether to continue or stop development of the candidate drug; and (3) understand the differences between those donors who experienced an efficacious response and those who did not, which is useful for the drug manufacturer to modify the compound to improve the response, and/or to appropriately select or monitor specific patients for clinical trials, and/or to develop an appropriate label that the candidate drug should only be used for patients with a certain genetic profile.

In another embodiment, the methods provided herein are used for issue-specific metabolism testing of pharmaceutical drugs, industrial chemicals, or other compounds or substances.

Chemicals and compounds are metabolized within the body primarily by CYP450 enzymes in the liver, but also by other enzymes and within other tissues. Degradation of a chemical or compound can terminate its efficacy, produce a metabolite that is more or less efficacious, terminate its toxicity or produce a metabolite that is more or less toxic, or increase the risk for drug-drug interactions. There is known and unknown genetic diversity in metabolizing enzymes, which can produce variations in exposure levels to drugs and metabolites, which can in turn affect efficacy and toxicity. Thus, extra-embryonic pluripotent or multipotent stem cells differentiated into liver cells from a panel of donors with genetic diversity is useful for identifying differences in the metabolism that can lead to population variance in efficacy and toxicity.

The methods provided herein enable analysis of pharmaceutical drugs, industrial chemicals, and other compounds and substances for variability in metabolism of chemical and compounds within specific cell types across genetically diverse donors, allowing the manufacturer to: (1) identify variability in metabolites and metabolism rates in specific cell types even if they occur with very low incidence (for example, in only 1% or 2% of the population); (2) determine whether to continue or stop development of chemical or compound; and (3) understand the differences in metabolism of the chemical or compound, which is useful for helping the drug manufacturer to modify the chemical or compound for more consistent metabolism across a population, and/or to appropriately select or monitor specific patients for clinical trials, and/or to develop an appropriate warning that the candidate drug should not be administered to patients with a certain genetic profile or to be aware of certain drug-drug interactions.

In another embodiment, the methods described herein are useful for identifying compounds, biologic agents, or other substances that produce consistent differentiation into specific tissue types across a genetically diverse population.

An emerging field of research is focused on identifying compounds, biologic agents or other substances that will induce differentiation of stem cells into specific tissue types. This often involves the manipulation of cellular mechanisms such as, but not limited to, gene expression, receptor signaling, or second messenger systems. Such compounds, agents or substances are being made commercially available for the differentiation of stem cells into specific tissue types for various applications including, but not limited to, efficacy and toxicity testing. Such compounds, agents or substances are also useful for developing a pharmaceutical agent for the endogenous repair of lost tissue due to diseases or injury, such as, but not limited to, stroke, spinal cord injury, neurodegenerative diseases, muscle atrophy, cardiovascular disease, cancer, diabetes, liver disease, and osteoporosis.

Given that there is remarkable genetic diversity in cellular mechanisms, it unlikely that such compounds, biologic agents, or other substances will produce consistent differentiation into specific tissue types (including, but not limited to, cardiomyocytes, hepatocytes, osteoblasts, myocytes, chondrocytes, epithelial cells, liver cells, pancreatic cells, neurons, embryoid bodies or similar three-dimensional structures) across a diverse population. A panel of extra-embryonic pluripotent or multipotent stem cells from genetically diverse donors is useful for testing substances for consistency of differentiation across the donor sample.

The methods provided herein enable analysis of differentiation produced by compounds or biologic agents to (1) determine the incidence of differentiation into a specific tissue type within a sample donor population, and (2) identify phenotypic or genetic traits in order to understand the differences between those donors samples that underwent appropriate differentiation and those that did not, which is useful for helping the manufacturer to modify the compound for more consistent differentiation across a population, and/or to appropriately select or monitor specific patients for clinical trials for the administration of a pharmaceutical agent for the endogenous repair of lost tissue due to diseases or injury.

In another embodiment, extra-embryonic pluripotent or multipotent stem cells are useful for determining the susceptibility of a genetically diverse population to various mutations of an infectious disease.

Of vital interest to scientists studying the risk of particular strains of infectious diseases (such as influenza) is the portion of the population that is likely to become infected, and the identity of any sub-populations that might be at particularly high risk.

However, although the scientific community has developed protocols to infect individual stem cells with a variety of diseases (See Robinton & Daley, "The Promise Of Induced Pluripotent Stem Cells In Research And Therapy", Nature 481, 295-305 (2012), the community has lacked an appropriate stem cell model of a genetically diverse population from which it can project infection rates in the population as a whole. This is demonstrated by recent attempts to model the mutations of avian flu that could lead to a pandemic which relied on an animal model (specifically, ferrets), rather than even a simple human-centric model (see Herfst, S. et al., "Airborne transmission of influenza A/H5N1 virus between ferrets", Science 336, 1534-1541 (2012)).

In this embodiment, extra-embryotic pluripotent or multipotent stem cells from a large number of donors representing a broad sample of individuals from a genetically diversified population of interest are distributed in solution into separate wells of a microtiter plate, and cultured appropriately. The cells are then exposed to the bacteria or virus known to cause the disease in question (where "cause" could refer to causality in at least one human, or causality in an animal species in the case of a zoonotic experiment), using standard protocols already established for that particular disease agent. For example, the exposure may be achieved via the delivery of a known concentration of the biological agent in solution by pipetting a predetermined amount into each well.

The wells are then sealed individually or collectively as appropriate, and the cells incubated for a specified period of time (usually 24 to 72 hours), according to the protocol for that particular agent. At that time, the infectious solution is separated from the cells, the cells are washed, and tests performed on each well individually, again according to the protocol.

The results of each well are then analyzed collectively to determine, for example: the overall portion of the population that has been infected; whether the infection rates are different for sub-populations, identified either phenotypically (such as race and gender) or genotypically (via the Whole Genome Sequences taken of each donor as described elsewhere herein).

In situations where the original infectious agent is hosted in an animal species, the experiment is repeated, but with the original infectious agent being replaced with the infectious agent isolated from the cells of individual donors successfully infected in the original experiment. In this way, scientists learn whether the agent has mutated in such a way that higher (or lower) rates of transmission between humans than between animals and humans (i.e., zoonotic transmission) are observed.

The example below is intended to further illustrate certain aspects of the methods described herein, and are not intended to limit the scope of the claims.

### EXAMPLE

### Use of extra-embryonic stem cells to test pharmaceutical compounds and industrial chemicals in vitro for toxicity

An experiment is conducted to determine whether extra-embryonic stem cells isolated from neonatal amniotic fluid will react similarly to known cytotoxins as compared to previously-derived control cells that have been validated as being predictive of *in vivo* toxicity (mouse ESC) and have been tested with known clinical toxicants (mouse ESC and human ESC).

### Test Subjects

For the human neonatal-derived cell lines, mothers of the neonates are tested for various pathogens by their OB/GYNs prior to delivery. Upon receipt, all samples are given a unique identifier associated with donor information and tissue source. Samples from human donors are processed to obtain up to 300 cell lines from amniotic fluid. Each cell line is expanded for cytotoxicity testing. Cells are frozen in multiple vials, with 1-2 M cells per vial. Prior to freezing, cells are tested for mycoplasma contamination. Cells are tested for growth and viability by Trypan blue exclusion or a similar method upon thawing.

Up to 300 donor samples of amniotic fluid cells, which are extra-embryonic pluripotent or multipotent stem cells derived from amniotic fluid are characterized by testing negative for CD-117, while testing positive for certain other cell surface markers (such as, but not limited to SSEA-4, SSEA-3, TRA-1-60, Tra-1-81) This cell is described in U.S. Patent No. 7,569,385.

### Cell Lines

A single mESC line (mouse ESC D3 (ATCC CRL-1934), is used to perform all preliminary calibrations, practice tests, etc. if needed. This same line is tested against the same seven concentrations of the same five test compounds as the test cells described below.

Further, a hESC line serves as a reference (Strehl 2007; Mehta et al., 2008). That is, results from the mESC and the test cells are compared to previously published results of cytotoxicity tests conducted on hESCs using a subset of the five compounds.

### Test Compounds

Five commonly available test compounds (pharmaceuticals and reference standards) with well-established toxic properties: 5-fluorouracil (5-FU, positive control), saccharin (negative control), all-trans retinoic acid (ATRA), 13-cis retinoic acid (13-cis-RA), and hydroxyurea (HU).

### Description of Cytotoxicity Test

A 10 day cytotoxicity test (Seiler and Spielman (2011) Nature Protocols 6: 961-978) is performed on each of up to 300 cell lines using a cell plating concentration based on cell growth observations from culturing the cells (initial assumption is 500 cells per well). Each of five compounds are tested at seven concentrations (plus a vehicle control), with five replicates per concentration. The cells are fed every 2-3 days. The concentration ranges of the five compounds are as follows:
5-FU, 0.0001 to 100 uM
ATRA, 13-cis-RA, and HU, 0.001 to 1000 uM
Saccharin, 0.01 to 10,000 uM

An MTT (3-(4,5-dimethyl-2-thiazolyl)-2,5-diphenyl-2H-tetrazolium bromide) cell viability assay is performed at the end of the study (day 10) to determine cell viability, and dose response curves are generated for each of the cell lines and compounds.

### ACQUISITION AND MAINTENANCE OF CELLS

1) Mouse embryonic cell line (mESC) is acquired from ATCC (mouse ESC D3 (ATCC CRL-1934)). mESC cells are cultured according to protocols described in Seiler and Spielman, 2011, (*supra*). After passaging post-thaw, mESC cells are cells are tested with AP Live Stain for comparison of pluripotency signal to other cell lines.
2) Isolation of Cell lines. Cell isolation will follow the protocol described in US patent number 7569385, which is summarized below.

Approximately 250 ml of fresh amniotic fluid is harvested from women undergoing caesarian delivery. Amniotic fluid is estimated to contain approximately 1-2×10⁴ live cells per ml. Upon arrival at the laboratory, the cells are pelleted in a clinical centrifuge and resuspended in 15 ml "MAFSC" medium. MAFSC medium is composed of low glucose Dulbecco Modified Eagle's Medium (GIBCO, Carlsbad, Calif.) and MCDB 201 medium (SIGMA, Saint Louis, Mo.) at a one to one ratio and contained 2% Defined Fetal Calf Serum (HYCLONE, Logan, Utah), 1× insulin-transferrin-selenium, linoleic-acid-bovine-serum-albumin (ITS+1, SIGMA), 1 nanomolar dexamethasone (Sigma), 100 µm ascorbic acid 2-phosphate (Sigma), 4 µm/ml gentamycin, 10 ng/ml of rhEGF (R&D Systems, Minneapolis, Minn.), 10 ng/ml rrPDGF-BB (R&D) and 10 ng/ml rhFGF-basic (R&D).

The wells of 6-well culture dishes are prepared for cell plating by coating for one hour at room temperature with 2.5 ml of fibronectin (stock of 10 µg fibronectin/ml of sterile water) immediately prior to cell plating. The fibronectin solution is removed prior to cell plating and the wells are not washed after removal of the fibronectin solution. The cells are then seeded in 2.5 ml of medium in each well. Around the time of seeding, cells are tested with AP Live Stain to determine if any pluripotent cells are present.

The cells in MAFSC culture appear under the inverted phase microscope as large suspension cells that divide on average once every 4 days, but cease dividing 8-12 days after seeding. The growth medium of MAFSC cultures is changed with complete MAFSC medium every two days making sure to not lose the suspended cells. After 8-10 days, small numbers of adherent cells emerge, which grow into large colonies of >10⁵ cells in 14-15 days. On average, 0-1 adherent colonies grow out per 2×10⁴ live cells seeded. Hence, a sample of 5 ml of fresh amniotic fluid gives rise to 3-5 adherent cell colonies, resulting in a single colony/clone in the majority of the wells of 6-well cell culture clusters.

Cells are transferred to successively larger fibronectin-coated flasks/vessels. To perform cell transfer, the cells are grown to a subconfluent state of approximately 40% confluence and are detached with 0.25% Trypsin-EDTA and replated at a 1:3 dilution under the same culture conditions.

Cells from amniotic fluid are purified by positive selection for SSEA4 expression and negative selection according to lack of CD117 expression by magnetic bead separation. Isolated cells are transferred to a tissue culture plate, MAFSC medium is then added to the isolated cells, and cells are grown in culture using standard cell culture conditions using MAFSC medium. After several passages, a sample of cells are taken for flow cytometry analysis of SSEA4 and CD117 expression to confirm the efficiency of the cell selection step. Cells will also be tested for pluripotency by the expression of Nanog and Oct-4 using flow cytometry. Cells are allowed to proliferate and expand in number to obtain sufficient quantities for cytotoxicity testing. Cells are maintained in MAFSC medium during the cytotoxicity test. Observations on morphological characteristics made during routine culturing of the cells at various stages prior to testing are noted.

### STEPS/PROCEDURES OF THE CYTOTOXICITY TESTS

Ideally, extra-embryonic stem cells and mESC cells are tested in parallel in the cytotoxicity test. If there is a large time delay between collecting cells from various donors, then cells can be stored frozen at similar population doubling/passage number, then thawed and expanded together in preparation for cytotoxicology testing.

A 10 day cytotoxicity test (Seiler and Spielman, 2011, (*supra*)) is performed on up to 300 cell lines using the optimized cell plating concentration determined based on observation of growth in culture (initial assumption is 500 cells per well). Each of five compounds are tested at seven concentrations (plus a vehicle control), with five replicates per concentration. The cells are fed every two to three days (remove approximately half of the media and replace with an equal volume of fresh medium including appropriate growth factors and test compounds).Concentration ranges of the compounds are as follow:
5-FU, 0.0001 to 100 uM
ATRA, 13-cis-RA, and HU, 0.001 to 1000 uM
Saccharin, 0.01 to 10,000 uM

An MTT assay is performed at the end of the study (day 10) to determine cell viability, and dose response curves are generated for each of the cell lines and compounds.

At the appropriate time (10 days from the application of the compound) for each well of the assay, portion of cells that remain viable is determined using a quantitative plate reader.

The resulting percentage is recorded in a set of Excel spreadsheets (as specified by the client), as well as analyze and display the results as follows

For each set of replicates (including any controls), the difference between the maximum and minimum percentage of cells that remain viable is determined.

The mean and standard error of the mean (SEM) of the replicates is computed, and will be used for graphical representation of the replicates.

On a single graph, the dose response curves are superimposed for all of the donors of a particular cell type and particular compound.

For each cell line, a graph that compares the compounds tested is generated.

Finally, responses from individual cell lines are grouped into sub-cohorts based on quantitative ranges of reaction, and gene association analyses are conducted to determine whether specific gene alleles, or combinations of gene alleles, are statistically associated with such differences in reaction.

The methods of the appended claims are not limited in scope by the specific methods described herein, which are intended as illustrations of a few aspects of the claims and any methods that are functionally equivalent are within the scope of this disclosure. Various modifications of the methods in addition to those shown and described herein are intended to fall within the scope of the appended claims. Further, while only certain representative methods, and aspects of these methods are specifically described, other methods and combinations of various features of the methods are intended to fall within the scope of the appended claims, even if not specifically recited. Thus, a combination of steps, elements, components, or constituents may be explicitly mentioned herein; however, all other combinations of steps, elements, components, and constituents are included, even though not explicitly stated.

## Claims

1. A method for predicting differential responses of humans to a biological or chemical substance comprising combining the substance with extra-embryonic stem cells from genetically different human donors *in vitro,* assaying for an effect on the cells, analyzing the effect on cells from each donor identifying differences in effects on the extra-embryonic stem cells from the genetically different donors, and comparing the differences across donors, wherein the comparison of the differences in effect from each donor predicts the differential responses that the substance will have on the humans.

2. The method of Claim 1 further comprising an intra-donor control for each donor, wherein the intra-donor control contains extra-embryonic stem cells from the same donor not combined with the substance.

3. The method of Claim 1 wherein the extra-embryonic stem cells are pluripotent or multipotent.

4. The method of Claim 1 wherein the extra-embryonic stem cells are expanded and differentiated prior to combination with the substance.

5. The method of Claim 4 wherein the donors are human neonates.

6. The method of Claim 4 wherein the extra-embryonic stem cells are from at least 20 genetically different donors, optionally wherein the extra-embryonic stem cells are from at least 300 genetically different donors.

7. The method of Claim 4 further comprising minimizing non-genetic inter-donor, cross-donor or intra-donor variability by selecting or excluding donors.

8. The method of Claim 4 further comprising minimizing non-genetic inter-donor, cross-donor or intra-donor variability by identifying differences in effects on the extra-embryonic stem cells from the genetically different donors utilizing statistical clustering techniques,
optionally wherein non-genetic inter-donor variability is minimized by selecting donors that are human neonates born in the same geographic location selected from the group consisting of city or region.

9. The method of Claim 7 wherein the donors are human neonates and non-genetic inter-donor variability is minimized by excluding donors meeting one or more criteria selected from the group consisting of known genetic abnormalities; evidence of newborn distress requiring intervention prior to or during fluid collection; undergone an experimental procedure or exposure to experimental product or ionizing radiation prior to delivery; fetal demise or major fetal anomaly; birth defect that may complicate the suitability of the amniotic fluid to contain stem cells; gestation not between 35-39 weeks of pregnancy; mother unwilling to disclose any and all use of medication/drugs/alcohol/tobacco or nicotine products during pregnancy; mother with complicated pregnancy; mother with major maternal medical illness associated with increased risk for adverse pregnancy outcome; mother with evidence of maternal infection with communicable disease that can transfer to neonate; category A infectious disease; mother with history of blood transfusion or receipt of blood product; evidence of low amniotic fluid; mother who used prescription drug, other than antiemetic, vitamin supplement, and delivery drug, during pregnancy; mother who used investigational drug during pregnancy lacking FDA approval; mother who consumed drug of abuse during pregnancy; and mother exposed to biological or chemical hazard over threshold known to cause chromosomal damage, birth defects, or chronic health impairment.

10. The method of Claim 7 wherein the donors are human neonates and non-genetic inter-donor variability is minimized by selecting donors born within a narrow window of time.

11. The method of Claim 7 wherein non-genetic variability is minimized by sequencing the DNA of all donors, aligning the sequences with the Human Reference Genome and excluding donors having DNA sequences that fail to align with the Human Reference Genome.

12. The method of Claim 7 wherein the extra-embryonic stem cells are expanded and differentiated under consistent production processes prior to combination with the substance to minimize non-genetic variability.

13. A method for predicting the prevalence or differential degree of reaction of a target population of humans to a biological or chemical substance comprising:
a. Identifying the target population,
b. Calculating a number of donors of the target population required from the desired level of discrimination,
c. Identifying known phenotypes of the target population associated with the response to the chemical or biological substance,
d. Providing extra-embryonic stem cells obtained from each donor for each phenotype,
e. Expanding and differentiating the stem cells capable of expansion and differentiation and replacing stem cells from any donor that fails to expand or differentiate with stem cells from a replacement donor having the same phenotype,
f. Combining the substance with the differentiated stem cells from each donor from each phenotype, assaying for an effect on the cells, comparing the effect on the cells from each donor from each phenotype with a control containing differentiated stem cells from the same donor not combined with the substance and
g. Conducting a statistical analysis of the effects, wherein a difference in the effect of the substance on the differentiated stem cells from the donors predicts the prevalence or differential degree of reaction within the target population.

14. An in vitro method of analyzing the results of two or more assays that compare and contrast the reactions of a cohort of stem cells to two or more biological or chemical substances, comprising:
a. adding one or more chemical or biological substances to a cohort of extra-embryonic stem cells or cells derived therefrom, from at least 10 genetically different donors for each of the two or more biological or chemical substances to be assayed and assaying for an effect on the cells;
b. quantitatively measuring the effect,
c. dividing the cohort into sub-cohorts by analysis of the quantitative measure using a statistical or quantitative technique that identifies statistical relationships between the sub-cohorts and/or the biological or chemical substances,
d. analyzing the statistical relationships of the two or more assays to compare and contrast the reactions of the sub-cohorts to the substances.

15. The method of Claim 14, further comprising:
a. Separately for each quantitative measure of step b, rank ordering members of the cohort from a lowest on that measure to a highest on that measure,
b. Determining the incremental increase of the quantitative measure of step c between a first member of the cohort and a second member of the cohort, by subtracting the quantitative measure of the first member from the quantitative measure of the second member,
c. Calculating the average incremental increase for each quantitative measure,
d. Defining as inflection points any instances where the increment between any two adjacent members of the cohort exceeds the average increment by more than a predetermined ratio, such as two-to-one,
e. Identifying as a separate sub-cohort any group of members that lie between inflection points, and
f. Conducting compare-and-contrast statistical analysis of the results from the various sub-cohorts,
optionally further comprising:
ai. Arraying the ordered ranking of members of step a in a matrix of dimensions, wherein each dimension corresponds to one measure,
bi. Subdividing each dimension into cohorts,
ci. identifying members of each cohort that occupy each intersection of cohorts on the matrix to create sub-cohorts, and
di. Conducting compare-and-contrast statistical analysis of the results from the various sub-cohorts.

## Patentansprüche

1. Verfahren zur Prognose unterschiedlicher Reaktionen von Menschen auf eine biologische oder chemische Substanz, das Folgendes umfasst: das Kombinieren der Substanz mit extraembryonalen Stammzellen aus genetisch unterschiedlichen menschlichen Spendern in vitro, das Untersuchen auf eine Wirkung auf die Zellen, das Analysieren der Wirkung auf Zellen von jedem Spender, wodurch Unterschiede der Wirkungen auf die extraembryonalen Stammzellen von den genetisch unterschiedlichen Spendern identifiziert werden, und das Vergleichen der Unterschiede über alle Spender, wobei der Vergleich der Wirkungsunterschiede von jedem Spender die unterschiedlichen Reaktionen prognostiziert, die die Substanz auf Menschen haben wird.

2. Verfahren nach Anspruch 1, das außerdem für jeden Spender eine Kontrolle im Spender umfasst, wobei die Kontrolle im Spender extraembryonale Stammzellen vom selben Spender enthält, die nicht mit der Substanz kombiniert wurden.

3. Verfahren nach Anspruch 1, wobei die extraembryonalen Stammzellen pluripotent oder multipotent sind.

4. Verfahren nach Anspruch 1, wobei die extraembryonalen Stammzellen vor der Kombination mit der Substanz expandiert und differenziert werden.

5. Verfahren nach Anspruch 4, wobei die Spender menschliche Neugeborene sind.

6. Verfahren nach Anspruch 4, wobei die extraembryonalen Stammzellen von zumindest 20 genetisch unterschiedlichen Spendern stammen, gegebenenfalls wobei die extraembryonalen Stammzellen von zumindest 300 genetisch unterschiedlichen Spendern stammen.

7. Verfahren nach Anspruch 4, das außerdem das Minimieren nicht genetischer Variabilität zwischen den Spendern, über die Spender oder im Spender durch das Auswählen oder Ausschließen von Spendern umfasst.

8. Verfahren nach Anspruch 4, das außerdem das Minimieren nicht genetischer Variabilität zwischen den Spendern, über die Spender oder im Spender umfasst, indem Unterschiede der Wirkungen auf die extraembryonalen Stammzellen von den genetisch unterschiedlichen Spendern unter Verwendung von statistischen Clusterverfahren identifiziert werden,
gegebenenfalls wobei die nicht genetische Variabilität zwischen den Spendern durch das Auswählen von Spendern minimiert wird, bei denen es sich um menschliche Neugeborene handelt, die am gleichen geographischen Ort geboren wurden, der aus der aus Stadt oder Region bestehenden Gruppe ausgewählt ist.

9. Verfahren nach Anspruch 7, wobei die Spender menschliche Neugeborene sind und die nicht genetische Variabilität zwischen den Spendern durch das Ausschließen von Spendern minimiert wird, die ein oder mehrere Kriterien erfüllen, die aus folgender Gruppe ausgewählt sind: bekannte genetische Abnormalitäten; Hinweis auf Leiden der Neugeborenen, die ein Eingreifen vor oder während der Fluidabnahme erfordern; Unterziehung eines experimentellen Vorgangs oder Aussetzung gegenüber einem experimentellen Produkt oder ionisierender Strahlung vor der Geburt; Totgeburt oder starke fötale Anomalie; Geburtsfehler, der die Eignung des Fruchtwassers, Stammzellen zu enthalten, verkomplizieren kann; Austragung nicht zwischen 35-39 Schwangerschaftswochen; Mutter nicht gewillt, einen Teil oder sämtliche Verwendung von Medikamenten/Drogen/Alkohol/Tabak- oder Nikotinprodukten während der Schwangerschaft offenzulegen; Mutter mit Schwangerschaftskomplikationen; Mutter mit schwerer maternaler medizinischer Krankheit, die mit einem erhöhten Risiko für einen nachteiligen Schwangerschaftsausgang verbunden ist; Mutter mit Hinweis auf maternale Infektion mit übertragbarer Krankheit, die auf das Neugeborene übertragen werden kann; Infektionskrankheit der Kategorie A; Mutter mit Vergangenheit von Bluttransfusionen oder Erhalt eines Blutprodukts; Hinweis auf wenig Fruchtwasser; Mutter, die während der Schwangerschaft ein verschreibungspflichtiges Arzneimittel einnahm, außer Antiemetika, Vitaminergänzungsmittel und Wehenfördermittel; Mutter, die während der Schwangerschaft Forschungspräparate ohne FDA-Zulassung einnahm; Mutter, die während der Schwangerschaft Drogen konsumierte; und Mutter, die einer biologischen oder chemischen Gefahr über dem bekannten Grenzwert für Chromosomenschäden, Geburtsfehler der chronischer Gesundheitsbeeinträchtigung ausgesetzt war.

10. Verfahren nach Anspruch 7, wobei die Spender menschliche Neugeborene sind und die nicht genetische Variabilität zwischen den Spendern durch das Auswählen von Spendern, die in einem engen Zeitfenster geboren wurden, minimiert wird.

11. Verfahren nach Anspruch 7, wobei die nicht genetische Variabilität durch das Sequenzieren der DNA von allen Spendern, das Abgleichen der Sequenzen mit dem menschlichen Referenzgenom und das Ausschließen von Spendern mit DNA-Sequenzen, die nicht mit dem menschlichen Referenzgenom abgeglichen werden können, minimiert wird.

12. Verfahren nach Anspruch 7, wobei die extraembryonalen Stammzellen unter gleich bleibenden Produktionsverfahren vor der Kombination mit der Substanz expandiert und differenziert werden, um die nicht genetische Variabilität zu minimieren.

13. Verfahren zur Prognose von Prävalenz oder unterschiedlichem Ausmaß einer Reaktion einer menschlichen Zielpopulation auf eine biologische oder chemische Substanz, das Folgendes umfasst:
a. das Identifizieren der Zielpopulation,
b. das Berechnen einer Anzahl von Spendern aus der Zielpopulation, die aus dem gewünschten Unterscheidungsausmaß erforderlich ist,
c. das Identifizieren bekannter Phänotypen der Zielpopulation, die mit der Reaktion auf die chemische oder biologische Substanz verbunden sind,
d. das Bereitstellen von extraembryonalen Stammzellen, die von jedem Spender jedes Phänotyps erhalten wurden,
e. das Expandieren und Differenzieren der Stammzellen, die zur Expansion und Differenzierung in der Lage sind, und das Ersetzen von Stammzellen von einem beliebigen Spender, die nicht expandieren oder differenzieren, durch Stammzellen von einem Ersatzspender mit dem gleichen Phänotyp,
f. das Kombinieren der Substanz mit den differenzierten Stammzellen von jedem Spender jedes Phänotyps, das Untersuchen auf eine Wirkung auf die Zellen, das Vergleichen der Wirkung auf die Zellen von jedem Spender jedes Phänotyps mit einer Kontrolle, die differenzierte Stammzellen desselben Spenders, die nicht mit der Substanz kombiniert wurden, enthält, und
g. das Durchführen einer statistischen Analyse der Wirkungen, wobei ein Wirkungsunterschied der Substanz auf die differenzierten Stammzellen von den Spendern die Prävalenz oder das unterschiedliche Ausmaß der Reaktion in der Zielpopulation prognostiziert.

14. In-vitro-Verfahren zur Analyse der Ergebnisse aus zwei oder mehr Tests, die die Reaktionen einer Gruppe von Stammzellen auf zwei oder mehr biologische oder chemische Substanzen vergleichen und gegenüberstellen, das Folgendes umfasst:
a. das Zusetzen von einer oder mehreren chemischen oder biologischen Substanzen zu einer Gruppe von extraembryonalen Stammzellen oder Zellen, die davon stammen, von zumindest 10 genetisch unterschiedlichen Spendern für jede der zwei oder mehr biologischen oder chemischen Substanzen zur Untersuchung und das Untersuchen auf eine Wirkung auf die Zellen;
b. das quantitative Messen der Wirkung,
c. das Teilen der Gruppe in Untergruppen durch die Analyse der quantitativen Messung unter Verwendung einer statistischen oder quantitativen Methode, die einen statistischen Zusammenhang zwischen den Untergruppen und/oder den biologischen oder chemischen Substanzen identifiziert,
d. das Analysieren der statistischen Zusammenhänge der zwei oder mehr Tests, um die Reaktionen der Untergruppen auf die Substanzen zu vergleichen und gegenüberzustellen.

15. Verfahren nach Anspruch 14, das außerdem Folgendes umfasst:
a. das separate für jede quantitative Messung von Schritt b Rangordnen der Gruppenmitglieder von einem niedrigsten dieser Messung zu einem höchsten dieser Messung,
b. das Bestimmen der inkrementellen Zunahme der quantitativen Messung von Schritt c zwischen einem ersten Mitglied der Gruppe und einem zweiten Mitglied der Gruppe durch das Subtrahieren der quantitativen Messung des ersten Mitglieds von der quantitativen Messung des zweiten Mitglieds,
c. das Berechnen der mittleren inkrementellen Zunahme für jede quantitative Messung,
d. das Definieren von beliebigen Fällen als Wendepunkte, in denen die Zunahme zwischen zwei beliebigen benachbarten Mitgliedern der Gruppe die mittlere Zunahme um mehr als ein vorbestimmtes Verhältnis, wie zwei-zu-eins, übersteigt,
e. das Identifizieren einer beliebigen Gruppe von Mitgliedern, die zwischen den Wendepunkten liegen, als separate Untergruppe und
f. das Durchführen einer statistischen Vergleichs- und Gegenüberstellungsanalyse der Ergebnisse aus den verschiedenen Untergruppen,
das gegebenenfalls außerdem Folgendes umfasst:
ai. das Anordnen des gereihten Rangs der Mitglieder aus Schritt a in einer Dimensionsmatrix, wobei jede Dimension einer Messung entspricht,
bi. das Unterteilen jeder Dimension in Gruppen,
ci. das Identifizieren von Mitgliedern jeder Gruppe, die jede Schnittmenge von Gruppen auf der Matrix besetzen, um Untergruppen zu bilden, und
di. das Durchführen einer statistischen Vergleichs- und Gegenüberstellungsanalyse der Ergebnisse aus den verschiedenen Untergruppen.

## Revendications

1. Procédé pour prédire des réponses différentielles d'humains à une substance biologique ou chimique comprenant la combinaison de la substance avec des cellules souches extra-embryonnaires de donneurs humains génétiquement différents *in vitro,* le test d'un effet sur les cellules, l'analyse de l'effet sur les cellules de chaque donneur en identifiant des différences d'effets sur les cellules souches extra-embryonnaires des donneurs génétiquement différents, et la comparaison des différences entre les donneurs, dans lequel la comparaison des différences d'effet de chaque donneur permet de prédire les réponses différentielles qu'aura la substance sur les humains.

2. Procédé selon la revendication 1, comprenant en outre un contrôle intra-donneur pour chaque donneur, dans lequel le contrôle intra-donneur contient des cellules souches extra-embryonnaires du même donneur non combinées avec la substance.

3. Procédé selon la revendication 1, dans lequel les cellules souches extra-embryonnaires sont pluripotentes ou multipotentes.

4. Procédé selon la revendication 1, dans lequel les cellules souches extra-embryonnaires sont soumises à une expansion et sont différenciées avant la combinaison avec la substance.

5. Procédé selon la revendication 4, dans lequel les donneurs sont des nouveau-nés humains.

6. Procédé selon la revendication 4, dans lequel les cellules souches extra-embryonnaires sont issues d'au moins 20 donneurs génétiquement différents, facultativement dans lequel les cellules souches extra-embryonnaires sont issues d'au moins 300 donneurs génétiquement différents.

7. Procédé selon la revendication 4, comprenant en outre la minimisation de la variabilité non génétique inter-donneurs, entre les donneurs ou intra-donneur en sélectionnant ou en excluant des donneurs.

8. Procédé selon la revendication 4, comprenant en outre la minimisation de la variabilité non génétique inter-donneurs, entre les donneurs ou intra-donneur en identifiant des différences d'effets sur les cellules souches extra-embryonnaires des donneurs génétiquement différents en utilisant des techniques de partitionnement statistique,
facultativement dans lequel la variabilité non génétique inter-donneurs est minimisée en sélectionnant des donneurs qui sont des nouveau-nés humains nés dans un même emplacement géographique sélectionné dans le groupe consistant en une ville ou une région.

9. Procédé selon la revendication 7, dans lequel les donneurs sont des nouveau-nés humains et la variabilité non génétique inter-donneurs est minimisée en excluant des donneurs qui satisfont à un ou plusieurs critères sélectionnés dans le groupe consistant en des anomalies génétiques connues ; des signes de détresse néonatale nécessitant une intervention avant ou pendant une collecte de liquide ; le fait d'avoir été soumise à une procédure expérimentale ou à une exposition à un produit expérimental ou à des rayonnements ionisants avant l'accouchement ; un décès foetal ou une anomalie foetale majeure ; une anomalie congénitale qui peut compliquer l'aptitude du liquide amniotique à contenir des cellules souches ; une gestation qui ne dure pas entre 35 et 39 semaines de grossesse ; une mère non disposée à divulguer toute utilisation de médicaments/consommation d'alcool/de tabac ou de produits contenant de la nicotine pendant la grossesse ; une mère présentant une grossesse compliquée ; une mère souffrant d'une affection médicale maternelle majeure associée à une augmentation du risque d'issue défavorable de la grossesse ; une mère présentant des signes d'infection maternelle par une maladie contagieuse qui peut être transmise au nouveau-né ; une maladie infectieuse de catégorie A ; une mère ayant des antécédents de transfusion sanguine ou d'administration d'un produit sanguin ; des signes d'une faible quantité de liquide amniotique ; une mère ayant utilisé un médicament délivré sur ordonnance autre qu'un antiémétique, un supplément vitaminique, et un médicament provoquant l'accouchement, pendant la grossesse ; une mère ayant pris un médicament expérimental pendant la grossesse qui n'a pas été autorisé par la FDA ; une mère ayant consommé de la drogue pendant la grossesse ; et une mère exposée à un danger biologique ou chimique excédant un seuil connu pour provoquer des dommages chromosomiques, des anomalies congénitales, ou un problème de santé chronique.

10. Procédé selon la revendication 7, dans lequel les donneurs sont des nouveau-nés humains et la variabilité non génétique inter-donneurs est minimisée en sélectionnant des donneurs nés dans un court intervalle de temps.

11. Procédé selon la revendication 7, dans lequel la variabilité non génétique est minimisée en séquençant l'ADN de tous les donneurs, en alignant les séquences avec le génome humain de référence et en excluant les donneurs dont les séquences d'ADN ne parviennent pas à être alignées avec le génome humain de référence.

12. Procédé selon la revendication 7, dans lequel les cellules souches extra-embryonnaires sont soumises à une expansion et sont différenciées selon des processus de production cohérents avant la combinaison avec la substance afin de minimiser la variabilité non génétique.

13. Procédé pour prédire la prévalence ou le degré de réaction différentiel d'une population d'humains cible à une substance biologique ou chimique comprenant :
a. l'identification de la population cible,
b. le calcul d'un nombre de donneurs de la population cible requis par rapport au niveau de discrimination souhaité,
c. l'identification de phénotypes connus de la population cible associés à la réponse à la substance chimique ou biologique,
d. la mise à disposition de cellules souches extra-embryonnaires obtenues à partir de chaque donneur pour chaque phénotype,
e. l'expansion et la différenciation des cellules souches capables d'expansion et de différenciation et le remplacement des cellules souches d'un donneur quelconque qui n'ont montré aucune expansion ou différenciation par les cellules souches d'un donneur de remplacement possédant le même phénotype,
f. la combinaison de la substance avec les cellules souches différenciées de chaque donneur de chaque phénotype, le test d'un effet sur les cellules, la comparaison de l'effet sur les cellules de chaque donneur de chaque phénotype avec un contrôle contenant des cellules souches différenciées du même donneur non combinées avec la substance et
g. la réalisation d'une analyse statistique des effets, dans laquelle une différence d'effet de la substance sur les cellules souches différenciées des donneurs permet de prédire la prévalence ou le degré de réaction différentiel au sein de la population cible.

14. Procédé *in vitro* d'analyse des résultats de deux essais ou plus qui comparent et mettent en contraste les réactions d'une cohorte de cellules souches vis-à-vis de deux substances biologiques ou chimiques ou plus, comprenant :
a. l'ajout d'une ou de plusieurs substances chimiques ou biologiques à une cohorte de cellules souches extra-embryonnaires, ou à des cellules dérivées de celles-ci, issues d'au moins 10 donneurs génétiquement différents pour chacune des deux substances biologiques ou chimiques ou plus devant être testées et le test d'un effet sur les cellules ;
b. la mesure quantitative de l'effet,
c. la division de la cohorte en sous-cohortes par une analyse de la mesure quantitative en utilisant une technique statistique ou quantitative qui identifie des relations statistiques entre les sous-cohortes et/ou les substances biologiques ou chimiques,
d. l'analyse des relations statistiques des deux essais ou plus afin de comparer et de mettre en contraste les réactions des sous-cohortes vis-à-vis des substances.

15. Procédé selon la revendication 14, comprenant en outre :
a. séparément pour chaque mesure quantitative de l'étape b, le classement ordonné des membres de la cohorte depuis le moins élevé pour cette mesure jusqu'au plus élevé pour cette mesure,
b. la détermination de l'augmentation incrémentale de la mesure quantitative de l'étape c entre un premier membre de la cohorte et un deuxième membre de la cohorte, en soustrayant la mesure quantitative du premier membre de la mesure quantitative du deuxième membre,
c. le calcul de l'augmentation incrémentale moyenne pour chaque mesure quantitative,
d. la définition, en tant que points d'inflexion, des cas dans lesquels l'incrément entre deux membres adjacents de la cohorte excède l'incrément moyen de plus d'un rapport prédéterminé, tel que deux pour un,
e. l'identification, en tant que sous-cohorte distincte, d'un quelconque groupe de membres qui se situe entre les points d'inflexion, et
f. la réalisation d'une analyse statistique de comparaison et de mise en contraste des résultats des diverses sous-cohortes, comprenant facultativement en outre :
ai. l'organisation du classement ordonné des membres de l'étape a dans une matrice de dimensions, où chaque dimension correspond à une seule mesure,
bi. la sous-division de chaque dimension en cohortes,
ci. l'identification des membres de chaque cohorte qui occupent chaque intersection des cohortes sur la matrice afin de créer des sous-cohortes, et
di. la réalisation d'une analyse statistique de comparaison et de mise en contraste des résultats des diverses sous-cohortes.
